# EUROPEAN PATENT SPECIFICATION

(11) **EP 4 450 610 B1**
(45) Date of publication and mention of the grant of the patent: **09.12.2026**
(21) Application number: 23746905.1
(22) Date of filing: 23.01.2023
(51) Int. Cl.: C12M 3/00

(54) **CULTURE DEVICE**
KULTURVORRICHTUNG
DISPOSITIF DE CULTURE

(30) Priority: 26.01.2022 JP 2022010203
(43) Date of publication of application: 23.10.2024
(73) Proprietor: PHC Holdings Corporation, Tokyo 100-8403 (JP)
(72) Inventor: OHTA, Akihiro, Toon-shi Ehime 791-0395 (JP); TEMMAN, Haruka, Toon-shi Ehime 791-0395 (JP); HITOMI, Tsugumasa, Toon-shi Ehime 791-0395 (JP); YAMASAKI, Naoyuki, Toon-shi Ehime 791-0395 (JP); HONDA, Kousuke, Toon-shi Ehime 791-0395 (JP)
(74) Representative: Grünecker Patent- und Rechtsanwälte PartG mbB
(86) International application number: PCT/JP2023/001960
(87) International publication number: WO 2023/145694

(56) References cited:
- WO-A1-2015/136794
- CN-A- 106 591 125
- CN-U- 212 560 302
- JP-A- 2013 066 435
- JP-A- 2013 523 086
- US-A- 4 336 329
- US-A1- 2013 052 101
- US-A1- 2016 010 049

## Description

### Technical Field

The present invention relates to a culture device.

### Background Art

In a culture device for incubating a culture such as a cell or a microorganism in a culture chamber, the culture chamber is humidified by heating a liquid stored in the culture chamber with a heater and evaporating the liquid. When the humidity in the culture chamber is increased early, the liquid is heated with a heater and evaporated, and at the same time, the liquid is evaporated with an ultrasonic vibrator (see, for example, Patent Literature 1, hereinafter, referred to as PTL 1).
A laboratory climatic cabinet, in particular a gassed incubator, is described in U.S. Patent Application Publication No. 2013/052101 A1. The gassed incubator has an interior enclosed by a housing and a steam generator arranged outside the interior, which is connected to the interior via a steam feed line. The steam generator is designed as an essentially unpressurized container having a base area and a steam region located above it, the base area being designed to accommodate a water reservoir and being provided with a heating device for heating the water reservoir. The steam feed line leaves the steam generator in the area of the steam region and an air feed line opens into the steam generator, in order to feed ambient air to the steam generator and to introduce air enriched with water steam via the steam feed line in essentially unpressurized form into the interior of the incubator.

### Citation List

### Patent Literature

PTL 1
Japanese Patent No. 5727187

### Summary of Invention

### Technical Problem

In order to achieve maintenance of an optimal culture environment in a culture device, there is a desire to early increase the humidity within the culture chamber, which has decreased due to door opening/closing, as in the culture device in PTL 1. In a case where the humidity in the culture chamber is increased early, the humidity in the culture chamber is likely to become relatively high due to overshoot, and condensation is likely to appear.

When water droplets due to condensation are generated within the culture chamber, bacteria are generated in the water droplets and adversely affect a culture. Accordingly, there is a desire to avoid the appearance of condensation in a culture device. In order to avoid the appearance of condensation in the culture chamber, it is also conceivable to use a humidity sensor for detecting the humidity in the culture chamber and to stop humidifying the culture chamber when the detection value of the humidity sensor becomes a target value. Having said that, the difference between the detection value of a humidity sensor and the actual humidity, that is, a detection error, varies for each humidity sensor, and further the difference between detection errors in humidity sensors is relatively large. That is, the detection value of a humidity sensor is not necessarily accurate. For this reason, when the humidity in the culture chamber becomes relatively high, condensation may appear within the culture chamber even when the detection value of a humidity sensor does not increase to a value at which condensation appears.

An object of the present disclosure is to achieve, in a culture device, both an increase in the humidity in the culture chamber early and prevention of condensation from appearing within the culture chamber.

### Solution to Problem

The object of the present disclosure is achieved by a culture device according to claim 1. A culture device according to the present disclosure includes: a culture chamber that accommodates a culture; a first vapor supplier that supplies vapor to the culture chamber by natural vaporization; a second vapor supplier that supplies vapor to the culture chamber by forced vaporization; a humidity sensor that detects humidity in the culture chamber; and a control device that operates the first vapor supplier and the second vapor supplier to humidify the culture chamber such that the humidity in the culture chamber becomes a target value. After the first vapor supplier and the second vapor supplier humidify the culture chamber until the humidity in the culture chamber becomes a determination value, the control device stops the second vapor supplier, and operates the first vapor supplier to humidify the culture chamber until the humidity in the culture chamber becomes the target value.

### Advantageous Effects of Invention

According to the present disclosure, it is possible to achieve, in a culture device, both an increase in the humidity in the culture chamber early and prevention of condensation from appearing within the culture chamber.

### Brief Description of Drawings

FIG. 1 is a schematic longitudinal section of a culture device in Embodiment 1 of the present disclosure as viewed from the right side;
FIG. 2 is a front view illustrating an outline of the inside of a culture chamber;
FIG. 3 is a flowchart that is executed in normal humidification control and rapid humidification control;
FIG. 4 is a flowchart that is executed in the rapid humidification control;
FIG. 5 is another example of the flowchart that is executed in the rapid humidification control; and
FIG. 6 is a flowchart that is executed in a second humidification mode.

### Description of Embodiments

### <Embodiment 1>

Hereinafter, a culture device according to Embodiment 1 of the present disclosure will be described with reference to the accompanying drawings. Note that, hereinafter, the side of culture device 1, which the user faces when using culture device 1, will be referred to as the front side (front surface side) of culture device 1, and the side opposite to the front side will be referred to as the rear side (rear surface side) of culture device 1. Further, the left and right sides of culture device 1 when viewed from the front will be referred to as the left side and right side of culture device 1, respectively. Further, the side of culture device 1, which is away from the surface on which culture device 1 is installed, will be referred to as the upper side (top surface side) of culture device 1, and the side opposite to the upper side will be referred to as the lower side (bottom surface side) of culture device 1.

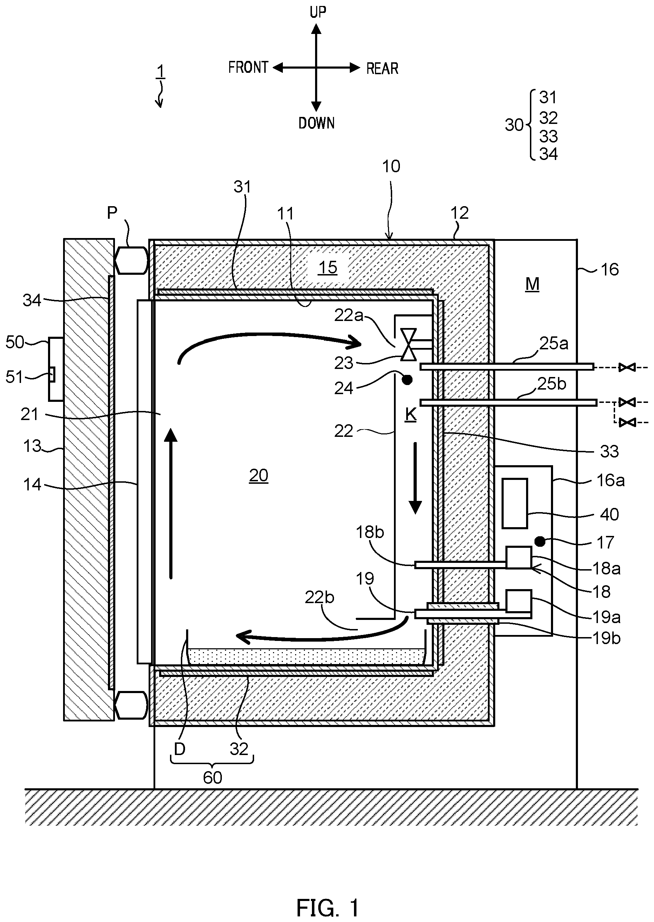

FIG. 1 is a schematic longitudinal section of culture device 1 as viewed from the right side. Culture device 1 is a device for incubating a culture such as a cell or a microorganism accommodated in culture chamber 20 formed inside housing 10 having a substantially box shape. Housing 10 includes inner box 11, outer box 12, outer door 13, and inner door 14.

Inner box 11 has a substantially box shape, includes culture chamber 20 on the inside thereof, and includes opening 21 of culture chamber 20 at the front surface thereof. Outer box 12 has a substantially box shape and covers a portion other than opening 21 on the outside of inner box 11. Inner box 11 and outer box 12 are formed of a metal plate. Heat insulation material 15 is disposed between inner box 11 and outer box 12.

Outer door 13 and inner door 14 open and close opening 21. Packing P is disposed at the outer edge of outer door 13.

Further, a plurality of heaters 30 for heating culture chamber 20 is disposed in housing 10. Each of the plurality of heaters 30 is formed in a plate shape. Specifically, the plurality of heaters 30 is each formed by disposing a cord heater (not illustrated) on a metal plate. The rated outputs of the plurality of heaters 30 may be different from each other or may be the same as each other.

On the outside of inner box 11, the plurality of heaters 30 includes: top-surface heater 31 disposed on the top surface of inner box 11; bottom-surface heater 32 disposed on the bottom surface of inner box 11; rear-surface heater 33 disposed on the rear surface of inner box 11; and side-surface heaters (not illustrated) disposed on the left and right side surfaces of inner box 11. Further, the plurality of heaters 30 includes outer-door heater 34 disposed at outer door 13.

Further, duct 22 that vertically extends on the inner rear surface of inner box 11 is disposed in culture chamber 20. Gas passage K is formed inside duct 22. In this gas passage K, circulation fan 23 is disposed. By activating circulation fan 23, air in culture chamber 20 is sucked in through suction port 22a formed in an upper portion of duct 22, and this air is blown out to culture chamber 20 through blow-out port 22b provided in a lower portion of duct 22. Thus, forced circulation of the air as indicated with the bold arrows is performed. Within duct 22, room temperature sensor 24 and gas supply devices 25a and 25b are disposed.

Room temperature sensor 24 detects the temperature in culture chamber 20. Specifically, room temperature sensor 24 is disposed in the vicinity of suction port 22a and detects the temperature of air sucked in through suction port 22a.

Gas supply devices 25a and 25b supply culture chamber 20 with an adjustment gas (for example, CO₂ gas, O₂ gas, and N₂ (nitrogen) gas) for adjusting the O₂ gas concentration and the CO₂ gas concentration in culture chamber 20.

Humidification tray D (a storage in the present disclosure) that stores a liquid (specifically water) that turns into vapor for humidification is installed between the lower portion of duct 22 and the bottom surface of inner box 11. Water stored in humidification tray D is sterilized by ultraviolet irradiation performed by a UV lamp (not illustrated).

The water stored in humidification tray D evaporates (natural vaporization) substantially in proportion to the difference between the saturated vapor pressure according to the temperature of the water and the vapor pressure of gas-phase water within culture chamber 20. As described above, humidification tray D constitutes a vapor supplier (the first vapor supplier in the present disclosure) that supplies vapor to culture chamber 20 by natural vaporization. In the case of this configuration, vapor is supplied to culture chamber 20 by natural vaporization, and thus, the amount of vapor to be supplied varies depending on the humidity in culture chamber 20.

In Embodiment 1, however, bottom-surface heater 32 (a warmer in the present disclosure) that warms the water stored in humidification tray D is provided, and humidification tray D and bottom-surface heater 32 are configured to constitute vapor supplier 60 (the first vapor supplier in the present disclosure) that supplies vapor to culture chamber 20 by natural vaporization. Here, bottom-surface heater 32 warms the water stored in humidification tray D to a temperature less than the boiling point, which is therefore called warming. The water stored in humidification tray D is warmed by bottom-surface heater 32 and is naturally vaporized. Even in the case of this configuration, vapor is supplied to culture chamber 20 by natural vaporization, and thus, the amount of vapor to be supplied varies depending on the humidity in culture chamber 20.

In a case where the humidity in culture chamber 20 as a target value is relatively high, humidification may be performed by using vapor supplier 60 that includes humidification tray D and bottom-surface heater 32, whereas in a case where the foregoing humidity is not relatively high, humidification may be performed by using a vapor supplier that includes only humidification tray D, without using bottom-surface heater 32 (without performing humidification).

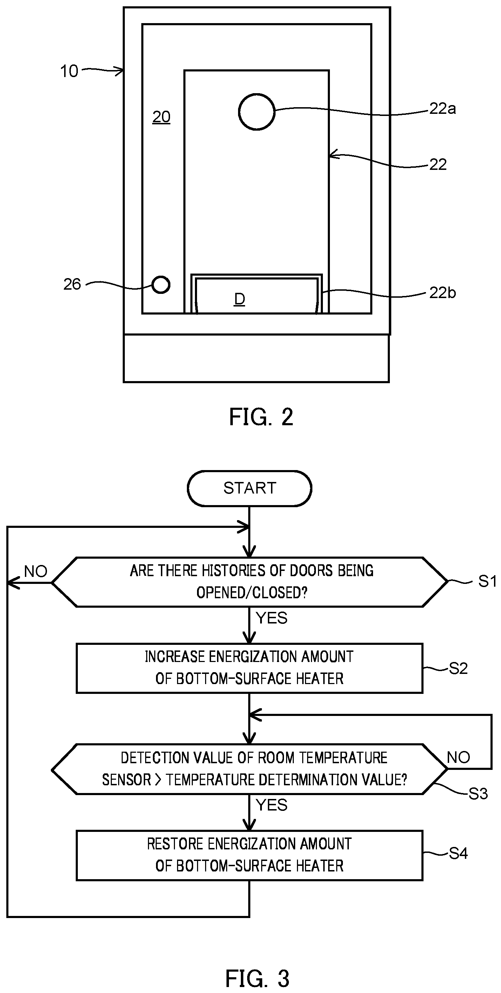

FIG. 2 is a front view illustrating an outline of the inside of culture chamber 20. Humidity sensor 26 is disposed on the inner rear surface of culture chamber 20. Humidity sensor 26 detects the humidity in culture chamber 20. In a lower portion of the inner rear surface of culture chamber 20, humidity sensor 26 is disposed on the left side of blow-out port 22b. It is needless to say that the position of humidity sensor 26 is not limited to the position illustrated in FIG. 2.

As illustrated in FIG. 1, the rear surface and bottom surface of outer box 12 of housing 10 are covered with cover 16. The space between the rear surface of outer box 12 and the cover forms mechanical room M for disposing various equipment therein. Mechanical room M is provided with electrical box 16a. Electrical box 16a houses control device 40.

In addition, culture device 1 further includes outside air sensor 17, vapor supply device 18, and dehumidification member 19. Outside air sensor 17 detects the ambient temperature of culture device 1.

Vapor supply device 18 supplies vapor to culture chamber 20. Vapor supply device 18 includes vapor generator 18a and vapor feeder 18b.

Vapor generator 18a is disposed in electrical box 16a, and includes a heater (not illustrated). Vapor generator 18a is supplied with water from a tank (not illustrated), in which water for vapor generation is stored, by a pump (not illustrated), and generates vapor by heating the water with the heater to evaporate the water. Vapor feeder 18b has a tubular shape, and supplies the vapor generated by vapor generator 18a to culture chamber 20.

In vapor supply device 18, the water supplied from the tank is heated by the heater and is subjected to forced vaporization. Here, the water supplied from the tank is heated to a temperature equal to or higher than the boiling point thereof and is evaporated, which is therefore called heating. In Embodiment 1, vapor generator 18a and vapor feeder 18b of vapor supply device 18 constitute a vapor supplier (the second vapor supplier in the present disclosure) that supplies vapor to culture chamber 20 by forced vaporization. Since vapor supply device 18 supplies vapor to culture chamber 20 by forced vaporization, the amount of vapor to be supplied is independent of the humidity in culture chamber 20, and is capable of supplying a desired amount of vapor.

For example, when control device 40 controls the pump, the amount per unit time of water to be supplied to vapor generator 18a is adjusted, and the amount per unit time of vapor to be supplied to culture chamber 20 is adjusted.

Dehumidification member 19 performs dehumidification such that the humidity within culture chamber 20 does not become too high. Dehumidification member 19 is made of metal and has a rod shape. A first end of dehumidification member 19 is located above humidification tray D within culture chamber 20. A second end of dehumidification member 19 is located within electrical box 16a. Cooling device 19a (for example, a Peltier element) for cooling dehumidification member 19 is attached to the second end of dehumidification member 19. Heat insulation material 19b is wound between the first end and second end of dehumidification member 19.

Control device 40 controls, based on the respective detection values of room temperature sensor 24 and outside air sensor 17, cooling device 19a such that the temperature of the first end of dehumidification member 19 is lower than the room temperature of culture chamber 20. Should the humidity in culture chamber 20 become relatively high, water droplets are generated only at the first end of dehumidification member 19. That is, it is possible to prevent water droplets from being generated at other portions of culture chamber 20 (for example, the inner surface of inner box 11) and at a culture.

Note that, dehumidification member 19 may be controlled based on the detection value of humidity sensor 26 in addition to the detection values of room temperature sensor 24 and outside air sensor 17. In this case, when the detection value of humidity sensor 26 is equal to or larger than a predetermined threshold defined in advance, cooling device 19a is controlled such that the temperature of the first end of dehumidification member 19 is lower than the room temperature of culture chamber 20. Thus, the humidity in culture chamber 20 can be increased early in a case where the detection value of humidity sensor 26 is lower than the predetermined threshold defined in advance. The predetermined threshold is a value lower than 100%, such as 90%.

Water droplets generated at the first end of dehumidification member 19 fall onto humidification tray D, and are sterilized by ultraviolet irradiation from the UV lamp. Accordingly, even in a case where water droplets are generated, it is possible to prevent the water droplets from adversely affecting a culture.

Culture device 1 receives instructions to start and stop culture device 1 and inputs of various setting values for culture chamber 20 through operating part 50 provided in outer door 13. The various setting values for culture chamber 20 are a set temperature, a set O₂ gas concentration, a set CO₂ gas concentration, and/or the like. Operator 50 includes a display that displays the state of culture device 1.

In a culture operation in which a culture is incubated in culture device 1, control device 40 controls circulation fan 23, gas supply devices 25a and 25b, the plurality of heaters 30, and/or the like such that the inside of housing 10 (culture chamber 20) has an atmosphere suitable for culture incubation.

Specifically, control device 40 starts the culture operation when the power supply of culture device 1 is turned on. In the culture operation, control device 40 controls the energization amounts of circulation fan 23 and the plurality of heaters 30 based on the detection value of room temperature sensor 24 such that the temperature in culture chamber 20 becomes a set temperature inputted into operating part 50. Each energization amount of top-surface heater 31, bottom-surface heater 32, rear-surface heater 33, the side surface heaters, and outer-door heater 34 is controlled so as to have a predetermined ratio defined in advance. Each predetermined ratio is derived in advance by experimentation or the like such that the temperature distribution in culture chamber 20 is uniform, and is stored in control device 40. Further, in the culture operation, control device 40 controls gas supply devices 25a and 25b such that the O₂ gas concentration and the CO₂ gas concentration in culture chamber 20 become the set O₂ gas concentration and the set CO₂ gas concentration, respectively.

When control device 40 starts the culture operation in response to turning-on of the power supply, control device 40 performs feedback control (for example, PID (Proportional Integral Differential) control) of the energization amounts of circulation fan 23 and the plurality of heaters 30 based on the detection value of room temperature sensor 24 such that the temperature in culture chamber 20 becomes a set temperature inputted into operating part 50. When the temperature in culture chamber 20 becomes stable at the set temperature inputted into operating part 50, the energization amounts of the plurality of heaters 30 become stable at energization amounts according to the outside air temperature, the set temperature, and each predetermined ratio. At this time, the energization amount of bottom-surface heater 32 becomes stable at a first energization amount.

When bottom-surface heater 32 generates heat in the culture operation, culture chamber 20 is humidified such that the water stored in humidification tray D evaporates and the humidity in culture chamber 20 becomes a target value. The target value is humidity suitable for incubating a culture, and is humidity in which no condensation appears within culture chamber 20. The target value is, for example, 95%. The actual humidity in culture chamber 20 becomes stable at a value determined by the temperature in culture chamber 20 and the temperature of the water stored in humidification tray D. The value of this stabilized actual humidity in culture chamber 20 will be referred to as the reached humidity value. The temperature of the water stored in humidification tray D eventually becomes stable at a constant value based on the energization amount of bottom-surface heater 32, even when the temperature temporarily changes due to, for example, replacement of water or the like. Thus, the reached humidity value becomes substantially equal to the target value. That is, when the energization amount of bottom-surface heater 32 becomes stable at the first energization amount described above, the actual humidity in culture chamber 20 becomes stable substantially at the target value. Note that, the temperature of the water stored in humidification tray D after the stabilization does not change in normal use, such as door opening/ closing in a short time, due to the height of the specific heat of the water.

Next, the control to make culture chamber 20 humidified, which control device 40 executes in the culture operation, will be described. Control device 40 selects one of normal humidification control, which makes culture chamber 20 humidified by activating the heater without activating vapor supply device 18, and rapid humidification control, which makes culture chamber 20 humidified by activating the heater and vapor supply device 18, and makes culture chamber 20 humidified such that the humidity in culture chamber 20 becomes the target value.

Control device 40 selects one of the normal humidification control and the rapid humidification control based on information inputted into operating part 50. The rapid humidification control is control to make culture chamber 20 humidified early in comparison with the normal humidification control.

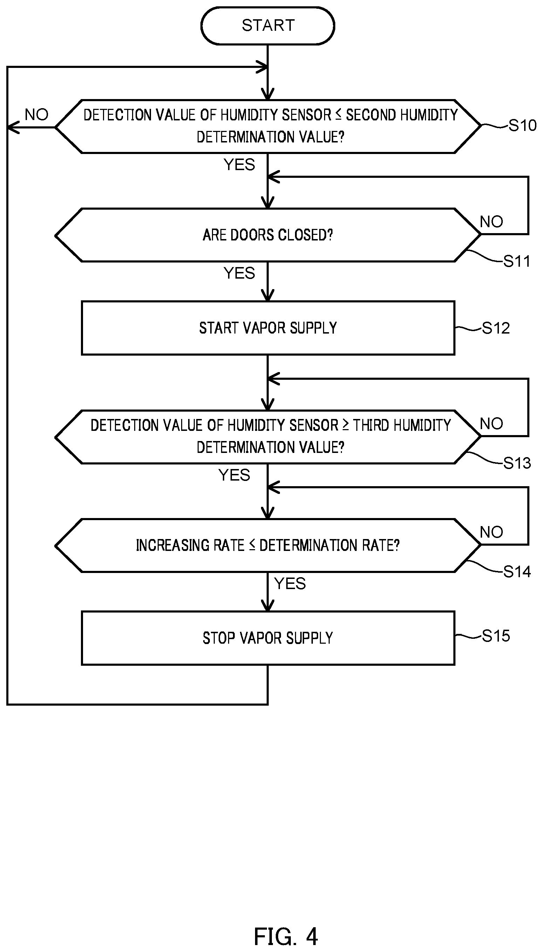

FIG. 3 is a flowchart that is executed whichever of the normal humidification control and the rapid humidification control is selected. FIG. 4 is a flowchart that is executed in parallel with the flowchart illustrated in FIG. 3 in a case where the rapid humidification control is selected. In a case where the normal humidification control is selected, control device 40 executes only the flowchart in FIG. 3.

Hereinafter, operations of culture device 1 when the normal humidification control is selected and control device 40 executes the flowchart in FIG. 3 will be described. In the culture operation, as described above, culture chamber 20 is humidified such that the humidity in culture chamber 20 becomes the target value by evaporation of the water stored in humidification tray D by means of heat generation by bottom-surface heater 32.

In S1, control device 40 determines whether there are histories of doors 13, 14 being opened/closed. Specifically, control device 40 determines whether doors 13, 14 are opened/closed between a current point in time and a point in time a predetermined time (for example, 20 minutes) before the current point in time, where the predetermined time is defined in advance. When doors 13, 14 open, high humidity air leaks out from culture chamber 20, which leads to a decrease in the humidity in culture chamber 20. The predetermined time is defined as a time sufficient for the humidity in culture chamber 20 to increase from a relatively low humidity (for example, 30% or lower) to a relatively high humidity (for example, 85% or higher) due to opening of doors 13, 14. Note that, control device 40 may determine in S1 whether or not it is within a predetermined time from the time when the power is turned on.

In a case where there are no histories of doors 13, 14 being opened/closed (NO in S1), control device 40 continues to control bottom-surface heater 32 with the first energization amount. In a case where there are histories of doors 13, 14 being opened/closed (YES in S1), on the other hand, control device 40 makes culture chamber 20 humidified by increasing the energization amount of bottom-surface heater 32 in S2.

Specifically, control device 40 sets the energization amount of bottom-surface heater 32 to a second energization amount larger than the first energization amount. Thus, the output of bottom-surface heater 32, and further the evaporation amount per unit time of the water stored in humidification tray D increase. Accordingly, in a case where the humidity in culture chamber 20 becomes relatively low due to opening of doors 13, 14, it is possible to humidify culture chamber 20 early. In a case where there are no histories of doors 13, 14 being opened/closed within the predetermined time, on the other hand, culture chamber 20 is humidified while the energization amount of bottom-surface heater 32 remains the first energization amount. That is, in a case where the humidity in culture chamber 20 is relatively high within the predetermined time, the energization amount of bottom-surface heater 32 is not increased. Thus, by increasing the energization amount of bottom-surface heater 32 when the humidity in culture chamber 20 is relatively low, it is possible to prevent condensation from appearing.

Further, unlike the first energization amount that changes according to the outside air temperature, the set temperature, and each predetermined ratio, the second energization amount is defined as a constant value in advance, which does not change according to the outside air temperature, the set temperature, and each predetermined ratio. Thus, the humidity in the culture chamber 20 can be stably humidified at an early stage without being affected by the outside air temperature and the set temperature.

Subsequently, control device 40 determines in S3 whether the detection value of room temperature sensor 24 is larger than a temperature determination value. The temperature determination value is actually measured by experimentation or the like, is defined in advance, and is stored in control device 40 in advance. The temperature determination value is defined as, for example, a value lower than the set temperature by a predetermined value (for example, 0.2) defined in advance. In a case where the temperature in culture chamber 20 becomes higher than the set temperature, the humidity may increase and condensation may appear in the process of the temperature decreasing to the set temperature. Accordingly, by performing determination based on the detection value of room temperature sensor 24, it is possible to humidify culture chamber 20 early, to prevent the humidity in culture chamber 20 from becoming too high, and further to prevent condensation from appearing.

Note that, in S3, control device 40 may perform determination of the detection value of humidity sensor 26 in addition to the determination of the detection value of room temperature sensor 24. Specifically, control device 40 determines whether the detection value of room temperature sensor 24 is equal to or less than the temperature determination value and the detection value of humidity sensor 26 is equal to or less than a first humidity determination value. The first humidity determination value is actually measured by experimentation or the like, is defined in advance, and is stored in control device 40 in advance. Further, as will be described below, the first humidity determination value is defined in view of a detection error in humidity sensor 26, which is the difference between the detection value of humidity sensor 26 and the actual humidity in culture chamber 20. The magnitude of a detection error in humidity sensor 26 varies for each humidity sensor used as humidity sensor 26. The first humidity determination value is defined as a value smaller than a value obtained by subtracting the magnitude of a detection error in humidity sensor 26 from the target value. Accordingly, the actual humidity in culture chamber 20 when the detection value of humidity sensor 26 is the first humidity determination value becomes a value smaller than the target value. The first humidity determination value is defined as, for example, 80%. Thus, it is possible to prevent condensation from appearing when control device 40 is executing S3.

In a case where the detection value of room temperature sensor 24 is equal to or lower than the temperature determination value (NO in S3), control device 40 continues to control bottom-surface heater 32 with the second energization amount. In a case where the temperature in culture chamber 20 increases and the detection value of room temperature sensor 24 is larger than the temperature determination value (YES in S3), on the other hand, control device 40 restores the energization amount of bottom-surface heater 32 in S4 and continues to make culture chamber 20 humidified.

Then, when the temperature in culture chamber 20 becomes the set temperature and the atmosphere in culture chamber 20 reaches a state suitable for incubation, the energization amount of bottom-surface heater 32 becomes stable at the first energization amount and the actual humidity in culture chamber 20 becomes stable at the reached humidity value, that is, substantially the target value, as described above.

In a case where the rapid humidification control is selected, on the other hand, control device 40 executes the flowcharts in FIGS. 3 and 4 in parallel. Control device 40 executes the flowchart in FIG. 3 in the same manner as described above. Hereinafter, operations of culture device 1 when control device 40 executes the flowchart in FIG. 4 will be described. At the start of the flowchart in FIG. 4, vapor supply device 18 is not activated.

In S10, control device 40 determines whether the detection value of humidity sensor 26 is equal to or less than a second humidity determination value. In the same manner as in the first humidity determination value, the second humidity determination value is actually measured by experimentation or the like and is defined in advance such that the second humidity determination value becomes a value smaller than a value obtained by subtracting the magnitude of a detection error in humidity sensor 26 from the target value, and is stored in control device 40 in advance. The second humidity determination value is defined as, for example, 80%. In a case where the detection value of humidity sensor 26 is larger than the second humidity determination value (NO in S10), control device 40 continues the state in which vapor supply device 18 is not activated.

When the time between a point in time when the power supply is turned on and a current point in time is relatively short or when doors 13, 14 are opened, on the other hand, the humidity in culture chamber 20 becomes relatively low. Thus, in a case where the detection value of humidity sensor 26 becomes equal to or less than the second humidity determination value (YES in S10), control device 40 determines in S11 whether doors 13, 14 are closed.

In a case where doors 13, 14 are open (NO in S11), control device 40 continues the state in which vapor supply device 18 is not activated. In a case where doors 13, 14 are closed (YES in S11), on the other hand, control device 40 starts supplying vapor in S12. Thus, it is possible to prevent vapor from leaking out from culture chamber 20 and the user or the like from being exposed to the vapor due to vapor supply starting when doors 13, 14 are open.

Control device 40 adjusts, based on the detection value of humidity sensor 26, the amount per unit time of the vapor to be supplied by vapor supply device 18 with feedback control (for example, PID control). In the PID control, control device 40 controls vapor supply device 18 such that the amount per unit time of the vapor to be supplied by vapor supply device 18 continuously decreases as the detection value of humidity sensor 26 becomes closer to the target value. Note that, control device 40 may execute, instead of the PID control, feedback control for changing the amount of vapor stepwise according to a detection value range of humidity sensor 26.

Further, the amount per unit time of the vapor to be supplied by vapor supply device 18 is larger than the evaporation amount per unit time of the water stored in humidification tray D in a case where bottom-surface heater 32 is controlled with the first energization amount. Thus, the humidity in the culture chamber 20 can be stably humidified at an early stage.

Subsequently, control device 40 determines in S13 whether the detection value of humidity sensor 26 is equal to or larger than a third humidity determination value. The third humidity determination value is actually measured by experimentation or the like, is defined in advance, and is stored in control device 40 in advance. Further, in the same manner as in the first humidity determination value, the third humidity determination value is defined based on a detection error in humidity sensor 26. Specifically, the third humidity determination value is defined as a value smaller than a value obtained by subtracting the magnitude of a detection error in humidity sensor 26 from the target value. Accordingly, the actual humidity in culture chamber 20 when the detection value of humidity sensor 26 is the third humidity determination value becomes a value smaller than the target value. The third humidity determination value is preferably a value smaller than the target value by at least 5% or more, which makes it possible to prevent the actual humidity within culture chamber 20 from exceeding the target value even when a detection error occurs in humidity sensor 26. Further, by configuring the third humidity determination value to be a value smaller than the target value by at least 10% or more, it is possible to prevent the actual humidity within culture chamber 20 from exceeding the target value even in the case of a delayed detection by humidity sensor 26 in addition to the case of a detection error in humidity sensor 26. In the present embodiment, the third humidity determination value is defined as, for example, 80%. The third humidity determination value may be the same value as or a different value from the first humidity determination value described above.

In a case where the detection value of humidity sensor 26 is smaller than the third humidity determination value (NO in S13), control device 40 operates vapor supply device 18 to continue vapor supply. In a case where the humidity in culture chamber 20 increases and the detection value of humidity sensor 26 becomes equal to or larger than the third humidity determination value (YES in S13), on the other hand, control device 40 executes S14. By stopping the vapor supply in S12 based on the third humidity determination value, which is a value smaller than the target value, in S13, the humidity in culture chamber 20 does not become too high and no condensation appears even in a case where a detection error occurs in humidity sensor 26. That is, it is possible to humidify culture chamber 20 without causing condensation to appear due to the influence of a detection error in humidity sensor 26.

Note that, the third humidity determination value is defined as becoming a value equal to or larger than the second humidity determination value. Thus, in a case when the humidity in culture chamber 20 decreases, it is possible to humidify culture chamber 20 without causing condensation to appear due to the influence of a detection error in humidity sensor 26.

Control device 40 determines in S14 whether the increasing rate, which is the increasing amount per unit time of the detection value of humidity sensor 26, is equal to or less than a determination rate. Control device 40 calculates the increasing rate based on the detection value of humidity sensor 26.

The present inventors have found as a result of research that, in a case where culture chamber 20 is humidified only with vapor evaporated from humidification tray D in a state in which vapor supply device 18 does not perform vapor supply, the increasing amount per unit time of the actual humidity in culture chamber 20 linearly decreases as the actual humidity in culture chamber 20 becomes closer to the reached humidity value. That is, the present inventors have found that in the above case, the increasing amount per unit time of the actual humidity in culture chamber 20 is in proportion to the difference between the reached humidity value and the actual humidity in culture chamber 20. The slope in this proportional relationship will be referred to as the humidification tray increasing rate coefficient. Further, the present inventors have found that the reached humidity value varies depending on the set temperature, whereas the humidification tray increasing rate coefficient does not significantly vary depending on the set temperature and varies to the extent of substantially ±20%.

Further, the increasing rate, which is the increasing amount per unit time of the detection value of humidity sensor 26, can be calculated without being affected by a detection error in humidity sensor 26. In addition, the increasing rate becomes equal to the increasing amount per unit time of the actual humidity in culture chamber 20 regardless of a detection error in humidity sensor 26. Further, in a case where there is no change in the temperature of the water stored in humidification tray D, for example, in a case of temporary doors opening/closing or the like, the reached humidity value is substantially equal to the target value. Accordingly, when the energization amount of bottom-surface heater 32 becomes stable at the first energization amount described above, the actual humidity in culture chamber 20 becomes stable substantially at the target value. That is, by calculating the increasing rate, it is possible to detect the difference between the actual humidity in culture chamber 20 and the target value indirectly without being affected by a detection error in humidity sensor 26.

Further, as described above, when the atmosphere in culture chamber 20 is stable in a state suitable for incubation, the actual humidity in culture chamber 20 becomes stable at the reached humidity value substantially equal to the target value at which no condensation appears within culture chamber 20. When culture chamber 20 is stable in a state suitable for incubation, no condensation appears in culture chamber 20.

Accordingly, when control device 40 controls the vapor supply by vapor supply device 18 based on the increasing rate such that the actual humidity in culture chamber 20 becomes equal to the target value, it is possible to humidify culture chamber 20 without causing condensation to appear due to the influence of a detection error in humidity sensor 26.

The determination rate in S14 is defined in advance, without taking a detection error in humidity sensor 26 into consideration, based on the vapor supply by vapor supply device 18, the foregoing reached humidity value actually measured by experimentation or the like, and the humidification tray increasing rate coefficient, and is stored in control device 40 in advance. Specifically, the determination rate is defined such that the actual humidity in culture chamber 20 when the increasing rate is the determination rate becomes humidity, which is higher than the actual humidity in culture chamber 20 when the detection value of humidity sensor 26 is the third humidity determination value, and which is lower than the target value.

For example, a case where the determination rate is defined as a value corresponding to when the actual humidity in culture chamber 20 is a value (for example, 85%) lower than the target value (95%) by 10%, where the target value is substantially equal to the reached humidity value, will be described. It has been found by experimentation or the like that, in a case where the actual humidity in culture chamber 20 is a value lower than the target value by 10%, the increasing amount per unit time of the actual humidity in culture chamber 20 due to evaporation of the water stored in humidification tray D is 1%/min based on the reached humidity value and the humidification tray increasing rate coefficient. Further, in this case, feedback control is performed such that the amount of the vapor to be supplied by vapor supply device 18 becomes 2%/min. Thus, the determination rate is defined as 3%/min, which is the sum of the increasing amount per unit time (1%/min) of the actual humidity in culture chamber 20 due to evaporation of the water stored in humidification tray D and the amount of the vapor to be supplied (2%/min) by vapor supply device 18.

Thus, even in a case where the humidity in culture chamber 20 is relatively high with the detection value of humidity sensor 26 exceeding the third humidity determination value, it is possible to increase the humidity in culture chamber 20 quickly without causing condensation to appear. Note that, by configuring the determination rate to be a value corresponding to a value (for example, 90%) lower than the target value by 5% or more, it is possible to prevent the actual humidity within culture chamber 20 from exceeding the target value even in the case of a delayed detection by humidity sensor 26.

Specific operations of control device 40 in S14 and thereafter will be described below.

In a case where the increasing rate is higher than the determination rate (NO in S14), control device 40 operates vapor supply device 18 to continue vapor supply.

Note that, in a case where the increasing rate is higher than the determination rate (NO in S14), control device 40 controls vapor supply device 18 in a manner different from that in S12. Specifically, control device 40 adjusts, based not on the detection value of humidity sensor 26, but on the increasing rate, the amount per unit time of the vapor to be supplied by vapor supply device 18 with feedback control. In the control of the amount of vapor, control device 40 may change the amount of vapor stepwise according to the range of the increasing rate, or may change the amount of vapor continuously according to the increasing rate as in the PID control. In the PID control, control device 40 controls vapor supply device 18 such that the amount per unit time of the vapor to be supplied by vapor supply device 18 decreases as the increasing rate becomes closer to zero.

For example, when the increasing rate is 6%RH/min, control device 40 may control vapor supply device 18 such that the amount of the vapor to be supplied by vapor supply device 18 becomes 4%RH/min, that is, 2/3 times 6%RH/min. Thus, by using a coefficient less than 1, the ratio of the amount of the vapor to be supplied by vapor supply device 18 to the increasing amount per unit time of the actual humidity in culture chamber 20 due to evaporation of the water stored in humidification tray D can be kept constant, and humidification can be performed stably up to the reached humidity value.

In particular, by using a coefficient equal to or less than 3/4, the ratio of the amount of the vapor to be supplied by vapor supply device 18 to the increasing amount per unit time of the actual humidity in culture chamber 20 due to evaporation of the water stored in humidification tray D can be kept to be equal to or less than 3:1. Thus, even when a slight error occurs in the amount of the vapor to be supplied by vapor supply device 18, it is possible to quickly increase the humidity in culture chamber 20 without causing condensation to appear.

In a case where the humidity in culture chamber 20 increases and the increasing rate is equal to or less than the determination rate (YES in S14), on the other hand, control device 40 stops the vapor supply in S15. Specifically, control device 40 stops the activation of vapor supply device 18.

Then, control device 40 activates, based on the flowchart in FIG. 3 executed in parallel with the flowchart in FIG. 4, bottom-surface heater 32 to humidify culture chamber 20. That is, in a case where the actual humidity in culture chamber 20 increases and the increasing rate is equal to or less than the determination rate (YES in S14), control device 40 makes culture chamber 20 humidified by activating bottom-surface heater 32 without activating vapor supply device 18. When the atmosphere in culture chamber 20 reaches a state suitable for incubation, the actual humidity in culture chamber 20 is maintained at the reached humidity value, that is, substantially the target value. At this time, bottom-surface heater 32 may be controlled by control device 40 to warm the water stored in humidification tray D such that the temperature of the water is changed according to the target value.

As described above, in the rapid humidification control, culture chamber 20 is humidified early since, in comparison with the normal humidification control, culture chamber 20 is humidified by the activation of bottom-surface heater 32 as well as vapor supply device 18 supplies vapor. Further, when the detection value of humidity sensor 26 becomes equal to or larger than the third humidity determination value and then the increasing rate becomes equal to or less than the determination rate, control device 40 makes culture chamber 20 humidified by activating bottom-surface heater 32 without activating vapor supply device 18. Thus, it is possible to prevent the humidity in culture chamber 20 from overshooting. Accordingly, it is possible to prevent condensation from appearing in culture chamber 20 due to the humidity in culture chamber 20 overshooting and becoming relatively high.

Further, control device 40 controls vapor supply device 18 such that the amount per unit time of the vapor to be supplied by vapor supply device 18 decreases as the detection value of humidity sensor 26 becomes closer to the target value. Accordingly, it is possible to surely prevent condensation from appearing in culture chamber 20 due to the humidity in culture chamber 20 overshooting and becoming relatively high.

Further, since the actual humidity in culture chamber 20 corresponding to the determination rate is closer to the reached humidity value (target value) than the actual humidity in culture chamber 20 corresponding to the third humidity determination value, the vapor supply by vapor supply device 18 can be performed until the difference between the actual humidity in culture chamber 20 and the target value becomes relatively small. Thus, it is possible to increase the humidity in culture chamber 20 early.

Further, in the present embodiment, the third humidity determination value is defined as 80%. Accordingly, even when the water stored in humidification tray D is depleted, the rapid humidification control is executed due to a decrease in the humidity in culture chamber 20, and thus, the humidity in culture chamber 20 can be increased early and can be kept at the third humidity determination value.

Note that, in S10, control device 40 may determine, in addition to whether the detection value of humidity sensor 26 is equal to or less than the second humidity determination value, whether the increasing rate is equal to or larger than a second determination rate. The second determination rate is defined such that the actual humidity in culture chamber 20 when the increasing rate is the second determination rate is between the actual humidity in culture chamber 20 when the detection value of humidity sensor 26 is the second humidity determination value and the actual humidity in culture chamber 20 when the detection value of humidity sensor 26 is the third humidity determination value. Thus, even in a case where the humidity in culture chamber 20 slightly decreases and does not become below the second humidity determination value, the vapor supply by vapor supply device 18 can be performed until the increasing rate becomes equal to or less than the determination rate. Thus, it is possible to increase the humidity in culture chamber 20 early.

Note that, in a case where the water stored in humidification tray D is cold water, condensation may appear at humidification tray D even when the humidity in culture chamber 20 is not a so high humidity (for example, approximately 50%).

The present inventors have found that, in a case where culture chamber 20 is humidified only with vapor evaporated from humidification tray D in a state in which vapor supply device 18 does not perform vapor supply and in a case where cold water is stored in humidification tray D, the humidity in culture chamber 20 becomes stable once at a second reached humidity value, which is lower than a first reached humidity value corresponding to the reached humidity value substantially equal to the target value, before the humidity in culture chamber 20 increases to the first reached humidity value, and that, when the water stored in humidification tray D is warmed, the humidity in culture chamber 20 increases from the second reached humidity value to the first reached humidity value and eventually becomes stable at the first reached humidity value. Further, the present inventors have found that, in a case where the rapid humidification control is selected, condensation appears on the outside of humidification tray D in a case where vapor supply by vapor supply device 18 is performed between when the humidity in culture chamber 20 increases from the second reached humidity value to the first reached humidity value and when the humidity in culture chamber 20 eventually becomes stable at the first reached humidity value. That is, the present inventors have found that, in a case where the rapid humidification control is selected, it is possible to prevent condensation from appearing on the outside of humidification tray D by performing vapor supply by vapor supply device 18 such that the humidity in culture chamber 20 does not exceed the second reached humidity value.

Nonetheless, since the second reached humidity value depends on the temperature of cold water which is stored in humidification tray D by the user, it has been difficult to grasp the second reached humidity value without measuring the temperature of the water stored in humidification tray D. In this respect, the present inventors have utilized the fact that, in a case where culture chamber 20 is humidified only with vapor evaporated from humidification tray D in a state in which vapor supply device 18 does not perform vapor supply, the increasing amount per unit time of the actual humidity in culture chamber 20 linearly decreases as the actual humidity in culture chamber 20 becomes closer to the reached humidity value as described above, and have configured such that vapor supply by vapor supply device 18 is performed such that the humidity in culture chamber 20 does not exceed the second reached humidity value, without grasping the second reached humidity value. Specifically, the present inventors have configured such that the third humidity determination value is a value (for example, 40%) much lower than the first and second reached humidity values, and the determination rate is, in the same manner as described above, 3%/min which is the determination rate when the amount of the vapor to be supplied by vapor supply device 18 is 2%/min (3%/min is a value corresponding to when the actual humidity in culture chamber 20 is a value lower than the second reached humidity value by 10%), and thus, control device 40 performs the determination according to S14 until the humidity in culture chamber 20 reaches from the third humidity determination value to the second reached humidity value. That is, when the difference between the actual humidity in culture chamber 20 and the second reached humidity value is accurately calculated based on the increasing rate regardless of a detection error in humidity sensor 26, culture chamber 20 can be humidified without the actual humidity in culture chamber 20 exceeding the second reached humidity value. That is, even in a case where cold water is stored in humidification tray D, it is possible to increase the humidity in culture chamber 20 without causing condensation to appear in culture chamber 20.

Note that, in a case where the humidity in culture chamber 20 exceeds the second reached humidity value due to the vapor supply by vapor supply device 18, water droplets adhere to the outside of humidification tray D, but when the humidity in culture chamber 20 slightly exceeds the second reached humidity value, the amount of water droplets may be small and water droplets may evaporate until the humidity in culture chamber 20 eventually becomes stable at the first reached humidity value. That is, no condensation appears on the outside of humidification tray D in the culture operation. In addition, as described above, by configuring the third humidity determination value to be relatively high, it is possible to keep the humidity in culture chamber 20 relatively high even when the water stored in humidification tray D is depleted. Further, the third humidity determination value is defined as 80% in the present embodiment. By configuring the third humidity determination value to be a value in a range of 50% to 85%, it is possible to increase the humidity in culture chamber 20 without causing condensation to appear in culture chamber 20, even in a case where cold water is stored in humidification tray D, even in a case where the water stored in humidification tray D is depleted, or even when a detection error occurs in humidity sensor 26.

### <Embodiment 2>

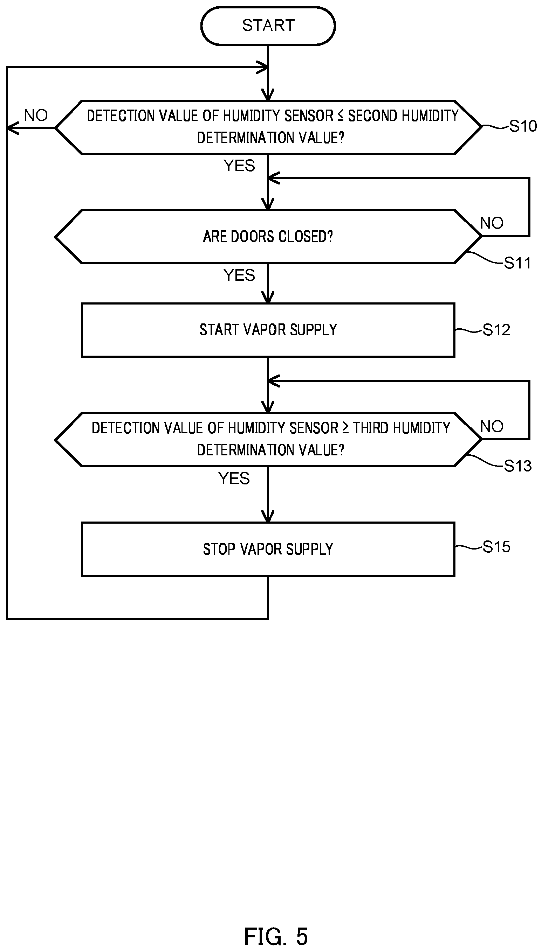

Next, culture device 1 according to Embodiment 2 of the present disclosure will be described with reference to FIG. 5. FIG. 5 is another example of the flowchart that is executed in the rapid humidification control. In the same manner as in FIG. 4, the flowchart illustrated in FIG. 5 is executed in parallel with the flowchart illustrated in FIG. 3 in a case where the rapid humidification control is selected.

Although the configuration of culture device 1 according to Embodiment 2 is the same as that of culture device 1 according to Embodiment 1 described above, the rapid humidification control according to Embodiment 2 partially differs from the rapid humidification control according to Embodiment 1. Specifically, the flowchart illustrated in FIG. 5 is substantially the same as the flowchart illustrated in FIG. 4, but is configured to proceed from S13 to S15 without performing S14 in the flowchart illustrated in FIG. 4. Accordingly, a description overlapping with the description of the flowchart illustrated in FIG. 4 will be omitted here.

S10 to S12 in the flowchart illustrated in FIG. 5 are the same as S10 to S13 in the flowchart illustrated in FIG. 4.

In a case where the detection value of humidity sensor 26 is smaller than the third humidity determination value in S13 (NO in S13), control device 40 operates vapor supply device 18 to continue vapor supply. In a case where the humidity in culture chamber 20 increases and the detection value of humidity sensor 26 becomes equal to or larger than the third humidity determination value (YES in S13), on the other hand, control device 40 executes S15. Here, by stopping the vapor supply in S12 based on the third humidity determination value, which is a value smaller than the target value, in S13, the humidity in culture chamber 20 does not become too high and no condensation appears, either, even in a case where a detection error occurs in humidity sensor 26. That is, it is possible to humidify culture chamber 20 without causing condensation to appear due to the influence of a detection error in humidity sensor 26.

Then, when the detection value of humidity sensor 26 becomes equal to or larger than the third humidity determination value (YES in S13), control device 40 stops the vapor supply by vapor supply device 18 in S15. That is, when the detection value of humidity sensor 26 becomes equal to or larger than the third humidity determination value, control device 40 makes culture chamber 20 humidified by activating bottom-surface heater 32 without activating vapor supply device 18.

After the vapor supply by vapor supply device 18 is stopped, control device 40 makes culture chamber 20 humidified by activating bottom-surface heater 32 based on the flowchart in FIG. 3 executed in parallel with the flowchart in FIG. 5. That is, control device 40 makes culture chamber 20 humidified by activating bottom-surface heater 32 without activating vapor supply device 18. When the atmosphere in culture chamber 20 reaches a state suitable for incubation, the actual humidity in culture chamber 20 is maintained at the reached humidity value, that is, substantially the target value. At this time, bottom-surface heater 32 may be controlled by control device 40 to warm the water stored in humidification tray D such that the temperature of the water is changed according to the target value.

As described above, in the rapid humidification control, culture chamber 20 is humidified early since, in comparison with the normal humidification control, culture chamber 20 is humidified by the activation of bottom-surface heater 32 as well as vapor supply device 18 supplies vapor. Further, after the detection value of humidity sensor 26 becomes equal to or larger than the third humidity determination value, control device 40 makes culture chamber 20 humidified by activating bottom-surface heater 32 without activating vapor supply device 18. Thus, it is possible to prevent the humidity in culture chamber 20 from overshooting. Accordingly, it is possible to prevent condensation from appearing in culture chamber 20 due to the humidity in culture chamber 20 overshooting and becoming relatively high.

### <Embodiment 3>

Next, culture device 1 according to Embodiment 3 of the present disclosure will be described mainly for parts different from those in culture device 1 according to Embodiment 1 described above.

Culture device 1 according to Embodiment 3 further includes inputter 51 into which the target value is inputted. Inputter 51 is, for example, a touch screen provided in operating part 50.

Further, control device 40 according to Embodiment 3 makes culture chamber 20 humidified by selecting one of a first humidification mode and a second humidification mode.

The first humidification mode is a mode in which control device 40 makes culture chamber 20 humidified by using vapor supplier 60 and vapor supply device 18 described above.

The second humidification mode is a mode in which control device 40 makes culture chamber 20 humidified by using vapor supply device 18 described above without using vapor supplier 60 described above. Specifically, the second humidification mode is a mode in which control device 40 makes culture chamber 20 humidified by activating vapor supply device 18 without activating bottom-surface heater 32 such that the detection value of humidity sensor 26 becomes the target value inputted into inputter 51. The target value in the second humidification mode is a value inputted into inputter 51 by the user, and is the humidity in culture chamber 20 that the user wishes.

In addition, no water is stored in humidification tray D in the second humidification mode. That is, in the second humidification mode, culture chamber 20 is humidified only with vapor supplied by vapor supply device 18. Note that, in the case of the second humidification mode, humidification tray D installed within culture chamber 20 may be taken out from culture chamber 20.

The selection of the first humidification mode or the second humidification mode is made, for example, according to the target value. For example, in a case where the humidity as the target value is 80 to 90%, the second humidification mode is selected and only vapor supply device 18 is used to humidify culture chamber 20. In a case where the humidity as the target value is equal to or larger than 90%, the first humidification mode is selected and vapor supplier 60 and vapor supply device 18 are used to humidify culture chamber 20. In the first humidification mode, the humidification control described in Embodiment 1 is performed.

At the time of switching from the first humidification mode to the second humidification mode, control device 40 may display indications, on the display of operating part 50, that a liquid stored in humidification tray D is discarded, humidification tray D is taken out from culture chamber 20, or the like. Further, at the time of switching from the second humidification mode to the first humidification mode, control device 40 may display indications, on the display of operating part 50, that a liquid is stored in humidification tray D, humidification tray D is installed in culture chamber 20, or the like.

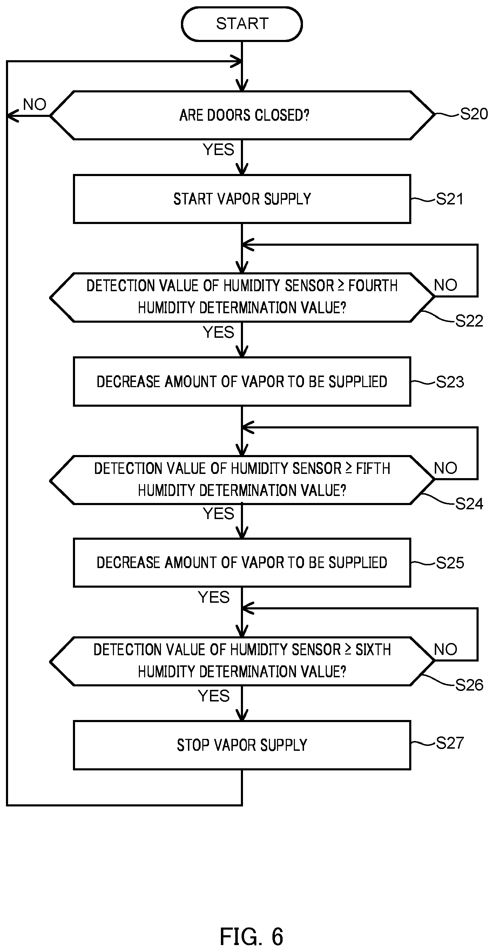

FIG. 6 is a flowchart that is executed in a case where the second humidification mode is selected. Hereinafter, operations of culture device 1 when control device 40 executes the flowchart in FIG. 6 will be described. At the start of the flowchart in FIG. 6, vapor supply device 18 is not activated.

Control device 40 determines in S20 whether doors 13, 14 are closed. In a case where doors 13, 14 are open (NO in S20), control device 40 continues the state in which vapor supply device 18 is not activated. In a case where doors 13, 14 are closed (YES in S20), on the other hand, control device 40 activates vapor supply device 18 and starts vapor supply in S21. Thus, it is possible to prevent vapor from leaking out from culture chamber 20 and the user or the like from being exposed to the vapor due to vapor supply starting when doors 13, 14 are open.

Control device 40 starts the vapor supply at a first supply amount that has been predetermined. The first supply amount is the amount per unit time of vapor to be supplied for increasing the humidity in culture chamber 20 early. The first supply amount is defined, for example, as being larger than the evaporation amount per unit time of the water stored in humidification tray D in a case where the water is stored in humidification tray D and bottom-surface heater 32 is controlled with the second energization amount.

Subsequently, control device 40 determines in S22 whether the detection value of humidity sensor 26 becomes equal to or larger than a fourth humidity determination value. The fourth humidity determination value is defined in advance so as to prevent the humidity in culture chamber 20 from overshooting and becoming relatively high in a case where the amount of vapor to be supplied is the first supply amount, and is stored in control device 40. When the target value for the second humidification mode is 95%, the fourth humidity determination value is, for example, a value corresponding to 95% of the target value, that is, 90.2%.

In a case where the detection value of humidity sensor 26 is smaller than the fourth humidity determination value (NO in S22), control device 40 continues the vapor supply with the first supply amount. In a case where the humidity in culture chamber 20 increases and the detection value of humidity sensor 26 becomes equal to or larger than the fourth humidity determination value (YES in S22), on the other hand, control device 40 decreases the amount of vapor to be supplied in S23.

Specifically, control device 40 performs the vapor supply with a second supply amount smaller than the first supply amount. The second supply amount is the amount per unit time of vapor to be supplied for preventing the humidity in culture chamber 20 from overshooting and becoming relatively high while increasing the humidity in culture chamber 20 early.

Subsequently, control device 40 determines in S24 whether the detection value of humidity sensor 26 becomes equal to or larger than a fifth humidity determination value. The fifth humidity determination value is defined in advance so as to be larger than the fourth humidity determination value and to prevent the humidity in culture chamber 20 from overshooting and becoming relatively high in a case where the amount of vapor to be supplied is the second supply amount, and is stored in control device 40. When the target value for the second humidification mode is 95%, the fifth humidity determination value is, for example, a value corresponding to 99.5% of the target value, that is, 94.5%.

In a case where the detection value of humidity sensor 26 is smaller than the fifth humidity determination value (NO in S24), control device 40 continues the vapor supply with the second supply amount. In a case where the humidity in culture chamber 20 increases and the detection value of humidity sensor 26 becomes equal to or larger than the fifth humidity determination value (YES in S24), on the other hand, control device 40 decreases the amount of vapor to be supplied in S25.

Specifically, control device 40 performs the vapor supply with a third supply amount smaller than the second supply amount. The third supply amount is the amount per unit time of vapor to be supplied for surely preventing the humidity in culture chamber 20 from overshooting and becoming relatively high.

Subsequently, control device 40 determines in S26 whether the detection value of humidity sensor 26 becomes equal to or larger than a sixth humidity determination value. The sixth humidity determination value is defined in advance so as to be larger than the fifth humidity determination value and to surely prevent the humidity in culture chamber 20 from overshooting and becoming relatively high in a case where the amount of vapor to be supplied is the third supply amount, and is stored in control device 40. When the target value for the second humidification mode is 95%, the sixth humidity determination value is, for example, a value corresponding to 99.95% of the target value, that is, 94.9%.

In a case where the detection value of humidity sensor 26 is smaller than the sixth humidity determination value (NO in S26), control device 40 continues the vapor supply with the third supply amount. In a case where the humidity in culture chamber 20 increases and the detection value of humidity sensor 26 becomes equal to or larger than the sixth humidity determination value (YES in S26), on the other hand, control device 40 stops the vapor supply in S27. At this time, the detection value of humidity sensor 26 is substantially equal to the target value.

As described above, control device 40 activates vapor supply device 18 such that the amount of vapor to be supplied gradually decreases as the humidity in culture chamber 20 becomes closer to the target value. Accordingly, it is possible to control the humidity in culture chamber 20 to be the target value inputted through inputter 51 while preventing the humidity in culture chamber 20 from overshooting.

### <Variations>

The present disclosure is not limited to the embodiments described thus far. Various variations to the embodiments and forms constructed by combining constituent elements in different embodiments are also within the scope of the present disclosure as long as they do not depart from the gist of the present disclosure.

For example, control device 40 may adjust the amount of vapor to be supplied by executing PID control instead of the flowchart in FIG. 6 in the second humidification mode.

Further, control device 40 may prevent condensation from occurring at dehumidification member 19 in the second humidification mode. In this case, control device 40 does not activate cooling device 19a. Thus, it is possible to control the humidity in culture device 1 to be a target value desired by the user accurately.

### Industrial Applicability

The present disclosure is suitably utilized as a culture device.

### Reference Signs List

1 Culture device
10 Housing
18 Vapor supply device
20 Culture chamber
26 Humidity sensor
30 Heater
32 Bottom-surface heater
40 Control device
51 Inputter
60 Vapor supplier

## Claims

1. A culture device, comprising:
a culture chamber (20) that accommodates a culture;
a first vapor supplier (60) adapted to supply vapor to the culture chamber by natural vaporization, wherein the vapor supplied by natural vaporization results from water stored in the first vapor supplier (60) and that evaporates substantially in proportion to a difference between a saturated vapor pressure according to a temperature of the water, which is at a temperature less than the water boiling point, and a vapor pressure of gas-phase water within the culture chamber (20);
a second vapor supplier (18) adapted to supply vapor to the culture chamber (20) by forced vaporization;
wherein the second vapor supplier (18) comprises a vapor generator (18a) and a vapor feeder (18b) adapted to supply vapor generated by the vapor generator (18a) to the culture chamber (20),
wherein the vapor generator (18a) is supplied with water from a tank in which water for vapor generation is stored, the water supplied from the tank being subjected to forced vaporization;
a humidity sensor (26) adapted to detect humidity in the culture chamber (20); and
a control device (40) adapted to make the culture chamber (20) humidified by the first vapor supplier (60) and/or the second vapor supplier (18) such that the humidity in the culture chamber (20) becomes a target value, wherein
the control device (40) is adapted to select one of rapid humidification control, which makes the culture chamber (20) humidified by the first vapor supplier (60) and the second vapor supplier (18), and normal humidification control, which makes the culture chamber (20) humidified by the first vapor supplier (60) without activating the second vapor supplier (18);
wherein the rapid humidification control is control to make the culture chamber (20) humidified early in comparison with the normal humidification control, and
wherein after the control device (40) is adapted to make the culture chamber (20) humidified by the first vapor supplier (60) and/or the second vapor supplier (18) until the humidity in the culture chamber (20) becomes a determination value lower than the target value, the control device (40) is adapted to stop the second vapor supplier (18) and make the culture chamber (20) humidified by the first vapor supplier (60) until the humidity in the culture chamber (20) becomes the target value.

2. The culture device according to claim 1, wherein
when a detection value of the humidity sensor (26) becomes equal to or larger than the determination value and then an increasing rate of the detection value of the humidity sensor (26) becomes equal to or less than a determination rate, the control device (40) is adapted to stop the second vapor supplier (18), and to operate the first vapor supplier (60) to humidify the culture chamber (20) until the humidity in the culture chamber (20) becomes the target value.

3. The culture device according to claim 1 or 2, wherein
the control device (40) is adapted to control the second vapor supplier (18) such that an amount per unit time of the vapor to be supplied by the second vapor supplier (18) decreases as a detection value of the humidity sensor (26) becomes closer to the target value.

4. The culture device according to any one of claims 1 to 3, further comprising an inputter (51) into which the target value is inputted, wherein
the control device (40) is adapted to make the culture chamber (20) humidified by selecting one of a first humidification mode and a second humidification mode, the first humidification mode being a mode in which the culture chamber (20) is humidified by using the first vapor supplier (60) and the second vapor supplier (18), the second humidification mode being a mode in which the culture chamber (20) is humidified by using the second vapor supplier (18) without using the first vapor supplier (60) such that a detection value of the humidity sensor (26) becomes the target value inputted into the inputter.

5. The culture device according to any one of claims 1 to 4, wherein
the first vapor supplier (60) includes:
a storage adapted to store a liquid that turns into the vapor; and
a warmer (32) adapted to warm the liquid that naturally vaporizes.

6. The culture device according to claim 5, wherein
the warmer (32) is adapted to warm the liquid such that the temperature of the liquid is changed according to the target value.

## Patentansprüche

1. Kulturvorrichtung, umfassend:
eine Kulturkammer (20), die eine Kultur aufnimmt;
eine erste Dampfzuführeinrichtung (60), die so eingerichtet ist, dass sie der Kulturkammer Dampf durch natürliche Verdampfung zuführt, wobei der durch natürliche Verdampfung zugeführte Dampf aus Wasser entsteht, das in der ersten Dampfzuführeinrichtung (60) gespeichert ist und das im Wesentlichen proportional zu einer Differenz zwischen einem Sättigungsdampfdruck entsprechend einer Temperatur des Wassers, die eine Temperatur unterhalb des Siedepunkts von Wasser ist, und einem Dampfdruck von gasförmigem Wasser innerhalb der Kulturkammer (20) verdampft;
eine zweite Dampfzuführeinrichtung (18), die so eingerichtet ist, dass sie der Kulturkammer (20) Dampf durch erzwungene Verdampfung zuführt;
wobei die zweite Dampfzuführeinrichtung (18) eine Dampferzeugungseinrichtung (18a) sowie eine Dampfeinleiteinrichtung (18b) umfasst, die so eingerichtet ist, dass sie von der Dampferzeugungseinrichtung (18a) erzeugten Dampf in die Kulturkammer (20) einleitet,
wobei der Dampferzeugungseinrichtung (18a) Wasser aus einem Tank zugeführt wird, in dem Wasser für Dampferzeugung gespeichert ist, und das aus dem Tank zugeführte Wasser erzwungener Verdampfung unterzogen wird;
einen Feuchtigkeitssensor (26), der so eingerichtet ist, dass er Feuchtigkeit in der Kulturkammer (20) erfasst; sowie
eine Steuervorrichtung (40), die so eingerichtet ist, dass sie bewirkt, dass die Kulturkammer (20) durch die erste Dampfzuführeinrichtung (60) und/oder die zweite Dampfzuführeinrichtung (18) so befeuchtet wird, dass die Feuchtigkeit in der Kulturkammer (20) einen Zielwert erreicht, wobei
die Steuervorrichtung (40) so eingerichtet ist, dass sie entweder eine Schnellbefeuchtungssteuerung, durch die die Kulturkammer (20) von der ersten Dampfzuführeinrichtung (60) und der zweiten Dampfzuführeinrichtung (18) befeuchtet wird, oder eine Normalbefeuchtungssteuerung auswählt, durch die die Kulturkammer (20) von der ersten Dampfzuführeinrichtung (60) befeuchtet wird, ohne die zweite Dampfzuführeinrichtung (18) zu aktivieren;
wobei die Schnellbefeuchtungssteuerung eine Steuerung ist, durch die die Kulturkammer (20) im Vergleich zur Normalbefeuchtungssteuerung früher befeuchtet wird, und
wobei die Steuervorrichtung (40) so eingerichtet ist, dass sie, nachdem sie bewirkt hat, dass die Kulturkammer (20) von der ersten Dampfzuführeinrichtung (60) und/oder der zweiten Dampfzuführeinrichtung (18) befeuchtet wird, bis die Feuchtigkeit in der Kulturkammer (20) einen Bestimmungswert erreicht, der niedriger als der Zielwert ist, die zweite Dampfzuführeinrichtung (18) zum Halten bringt und bewirkt, dass die Kulturkammer (20) von der ersten Dampfzuführeinrichtung (60) befeuchtet wird, bis die Feuchtigkeit in der Kulturkammer (20) den Zielwert erreicht.

2. Kulturvorrichtung nach Anspruch 1, wobei
die Steuervorrichtung (40) so eingerichtet ist, dass sie, wenn ein Erfassungswert des Feuchtigkeitssensors (26) den Bestimmungswert erreicht oder übersteigt, und anschließend eine Anstiegsrate des Erfassungswertes des Feuchtigkeitssensors (26) die Bestimmungsrate erreicht oder unterschreitet, die zweite Dampfzuführeinrichtung (18) zum Halten bringt und die erste Dampfzuführeinrichtung (60) so betreibt, dass sie die Kulturkammer (20) befeuchtet, bis die Feuchtigkeit in der Kulturkammer (20) den Zielwert erreicht.

3. Kulturvorrichtung nach Anspruch 1 oder 2, wobei
die Steuervorrichtung (40) so eingerichtet ist, dass sie die zweite Dampfzuführeinrichtung (18) so steuert, dass eine pro Zeiteinheit durch die zweite Dampfzuführeinrichtung (18) zugeführte Menge des Dampfes abnimmt, wenn ein Erfassungswertes des Feuchtigkeitssensors (26) sich dem Zielwert nähert.

4. Kulturvorrichtung nach einem der Ansprüche 1 bis 3, die des Weiteren eine Eingabevorrichtung (51) umfasst, in die der Zielwert eingegeben wird, wobei
die Steuervorrichtung (40) so eingerichtet ist, dass sie bewirkt, dass die Kulturkammer (20) befeuchtet wird, indem ein erster Befeuchtungsmodus oder ein zweiter Befeuchtungsmodus ausgewählt wird, wobei der erste Befeuchtungsmodus ein Modus ist, in dem die Kulturkammer (20) unter Einsatz der ersten Dampfzuführeinrichtung (60) und der zweiten Dampfzuführeinrichtung (18) befeuchtet wird, und der zweite Befeuchtungsmodus ein Modus ist, in dem die Kulturkammer (20) unter Einsatz der zweiten Dampfzuführeinrichtung (18) befeuchtet wird, ohne die erste Dampfzuführeinrichtung (60) einzusetzen, so dass ein Erfassungswert des Feuchtigkeitssensors (26) den in die Eingabevorrichtung eingegebenen Zielwert erreicht.

5. Kulturvorrichtung nach einem der Ansprüche 1 bis 4, wobei die erste Dampfzuführeinrichtung (60) aufweist:
eine Speichereinrichtung, die so eingerichtet ist, dass sie eine Flüssigkeit speichert, die sich in den Dampf verwandelt; und
eine Erwärmungseinrichtung (32), die so eingerichtet ist, dass sie die Flüssigkeit erwärmt, die natürlich verdampft.

6. Kulturvorrichtung nach Anspruch 5, wobei
die Erwärmungseinrichtung (32) so eingerichtet ist, dass sie die Flüssigkeit so erwärmt, dass die Temperatur der Flüssigkeit entsprechend dem Zielwert geändert wird.

## Revendications

1. Dispositif de culture, comprenant :
une chambre de culture (20) qui contient une culture ;
un premier module de fourniture de vapeur (60) conçu pour fournir de la vapeur à la chambre de culture par vaporisation naturelle, dans lequel la vapeur fournie par vaporisation naturelle provient d'une eau stockée dans le premier module de fourniture de vapeur (60) et qui s'évapore sensiblement proportionnellement à une différence entre une pression de vapeur saturante en fonction d'une température de l'eau, qui est à une température inférieure au point d'ébullition de l'eau, et une pression de vapeur d'une eau en phase gazeuse à l'intérieur de la chambre de culture (20) ;
un second module de fourniture de vapeur (18) conçu pour fournir de la vapeur à la chambre de culture (20) par vaporisation forcée ;
dans lequel le second module de fourniture de vapeur (18) comprend un générateur de vapeur (18a) et un alimentateur de vapeur (18b) conçu pour fournir de la vapeur générée par le générateur de vapeur (18a) à la chambre de culture (20),
dans lequel le générateur de vapeur (18a) est alimenté en eau à partir d'un réservoir dans lequel est stockée de l'eau destinée à la génération de vapeur, l'eau fournie par le réservoir étant soumise à une vaporisation forcée ;
un capteur d'humidité (26) conçu pour détecter l'humidité dans la chambre de culture (20) ; et
un dispositif de commande (40) conçu pour humidifier la chambre de culture (20) à l'aide du premier module de fourniture de vapeur (60) et/ou du second module de fourniture de vapeur (18) de telle sorte que l'humidité dans la chambre de culture (20) devienne égale à une valeur cible, dans lequel
le dispositif de commande (40) est conçu pour sélectionner l'une parmi une commande d'humidification rapide, qui humidifie la chambre de culture (20) à l'aide du premier module de fourniture de vapeur (60) et du second module de fourniture de vapeur (18), et une commande d'humidification normale, qui humidifie la chambre de culture (20) à l'aide du premier module de fourniture de vapeur (60) sans activer le second module de fourniture de vapeur (18) ;
dans lequel la commande d'humidification rapide est une commande visant à humidifier la chambre de culture (20) précocement par comparaison avec la commande d'humidification normale, et
dans lequel après que le dispositif de commande (40) est conçu pour humidifier la chambre de culture (20) à l'aide du premier module de fourniture de vapeur (60) et/ou du second module de fourniture de vapeur (18) jusqu'à ce que l'humidité dans la chambre de culture (20) devienne égale à une valeur de détermination inférieure à la valeur cible, le dispositif de commande (40) est conçu pour arrêter le second module de fourniture de vapeur (18) et humidifier la chambre de culture (20) à l'aide du premier module de fourniture de vapeur (60) jusqu'à ce que l'humidité dans la chambre de culture (20) devienne égale à la valeur cible.

2. Dispositif de culture selon la revendication 1, dans lequel
quand une valeur de détection du capteur d'humidité (26) devient supérieure ou égale à la valeur de détermination et qu'ensuite une vitesse d'augmentation de la valeur de détection du capteur d'humidité (26) devient inférieure ou égale à une vitesse de détermination, le dispositif de commande (40) est conçu pour arrêter le second module de fourniture de vapeur (18), et pour actionner le premier module de fourniture de vapeur (60) pour humidifier la chambre de culture (20) jusqu'à ce que l'humidité dans la chambre de culture (20) devienne égale à la valeur cible.

3. Dispositif de culture selon la revendication 1 ou 2, dans lequel
le dispositif de commande (40) est conçu pour commander le second module de fourniture de vapeur (18) de telle sorte qu'une quantité par unité de temps de la vapeur à fournir par le second module de fourniture de vapeur (18) diminue à mesure qu'une valeur de détection du capteur d'humidité (26) s'approche de la valeur cible.

4. Dispositif de culture selon l'une quelconque des revendications 1 à 3, comprenant en outre un module d'entrée (51) dans lequel est entrée la valeur cible, dans lequel
le dispositif de commande (40) est conçu pour humidifier la chambre de culture (20) en sélectionnant l'un parmi un premier mode d'humidification et un second mode d'humidification, le premier mode d'humidification étant un mode dans lequel la chambre de culture (20) est humidifiée en utilisant le premier module de fourniture de vapeur (60) et le second module de fourniture de vapeur (18), le second mode d'humidification étant un mode dans lequel la chambre de culture (20) est humidifiée en utilisant le second module de fourniture de vapeur (18) sans utiliser le premier module de fourniture de vapeur (60) de telle sorte qu'une valeur de détection du capteur d'humidité (26) devienne égale à la valeur cible entrée dans le module d'entrée.

5. Dispositif de culture selon l'une quelconque des revendications 1 à 4, dans lequel
le premier module de fourniture de vapeur (60) comporte :
un stockage conçu pour stocker un liquide qui devient la vapeur ; et
un réchauffeur (32) conçu pour chauffer le liquide qui se vaporise naturellement.

6. Dispositif de culture selon la revendication 5, dans lequel
le réchauffeur (32) est conçu pour chauffer le liquide de telle sorte que la température du liquide soit modifiée en fonction de la valeur cible.
